(19) Europäisches Patentamt
European Patent Office
Office européen des brevets

(11) Veröffentlichungsnummer: **0 261 478 B1**

# (12) EUROPÄISCHE PATENTSCHRIFT

(45) Veröffentlichungstag der Patentschrift: **24.04.91**

(51) Int. Cl.$^5$: **C07D 413/14**, C07D 495/04, A61K 31/415, A61K 31/44, A61K 31/42

(21) Anmeldenummer: **87112928.4**

(22) Anmeldetag: **04.09.87**

## (54) Neue Derivate von 4,5-Dihydro-oxazolen, Verfahren zu ihrer Herstellung und ihre Verwendung.

(30) Priorität: **10.09.86 AT 2437/86**

(43) Veröffentlichungstag der Anmeldung: **30.03.88 Patentblatt 88/13**

(45) Bekanntmachung des Hinweises auf die Patenterteilung: **24.04.91 Patentblatt 91/17**

(84) Benannte Vertragsstaaten: **AT BE CH DE ES FR GB GR IT LI LU NL SE**

(56) Entgegenhaltungen:
**EP-A- 0 005 129**
**EP-A- 0 173 664**
**EP-A- 0 201 094**
**EP-A- 0 234 485**

(73) Patentinhaber: **Hafslund Nycomed Pharma Aktiengesellschaft**
**St. Peter-Strasse 25**
**A-4021 Linz(AT)**

(72) Erfinder: **Binder, Dieter, Dr.**
**Sieveringerstrasse 207**
**A-1190 Wien(AT)**
Erfinder: **Rovenszky, Franz, Dr.Dipl.-Ing.**
**Lagerhausstrasse 5/8**
**A-2460 Bruck a.d. Leitha(AT)**
Erfinder: **Ferber, Hubert, Dr.**
**Ahornweg 24**
**A-4052 Ansfelden(AT)**

(74) Vertreter: **Kunz, Ekkehard, Dr.**
**Chemie Holding AG Patentwesen St. Peter-Strasse 25**
**A-4021 Linz(AT)**

**EP 0 261 478 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Derivate von 4,5-Dihydro-oxazolen, ein Verfahren zu ihrer Herstellung und ihre Verwendung.

Substituierte Pyridylsulfinylbenzimidazole und Pryidylsulfinylthienoimidazole mit einer Hemmwirkung auf die Magensäuresekretion sind bekannt und beispielsweise in den EP-Anmeldeschriften Nr. 0 005 129, 0 173 664, 0 201 094 und 0 234 485 beschrieben. Da jedoch das pharmakologische Wirkprofil dieser Substanzen noch nicht ausgetestet ist und es daher nicht klar ist, ob sich diese Substanzen in der Humantherapie auch wirklich verwenden lassen, besteht weiterhin ein Bedarf nach neuen Verbindungen mit magensaftsekretionshemmender Wirkung.

Gegenstand der Erfindung sind demnach neue 4,5-Dihydro-oxazole der allgemeinen Formel

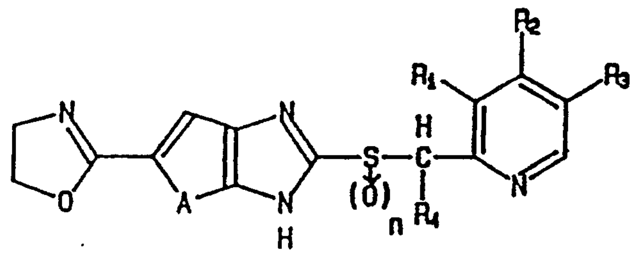

I

in der
A -CH=CH- oder -S-,
$R_1$, $R_3$ und $R_4$ unabhängig voneinander Wasserstoff oder ($C_1$-$C_4$)-Alkyl,
$R_2$ Wasserstoff oder (C1-C4)-Alkoxy und
n O oder 1 bedeuten
und Verfahren zu ihrer Herstellung, pharmazeutische Präparate, die diese Verbindungen enthalten, sowie ihre Verwendung.

Der in dieser Beschreibung verwendete Ausdruck "(C1-C4)-Alkyl" bezeichnet geradkettige oder verzweigte gesättigte Kohlenwasserstoffgruppen mit 1-4 Kohlenstoffatomen, wie z.B. Methyl, Ethyl, Propyl, Isopropyl, Butyl, tert. Butyl. Der Ausdruck (C1-C4)-Alkoxy bezieht sich auf Hydrocarbonoxygruppen mit 1-4 Kohlenstoffatomen, z.B. Methoxy, Ethoxy, Propyloxy, Isopropyloxy, Butyloxy, tert. Butyloxy.

In einer bevorzugten Gruppe von Verbindungen der Formel 1 bedeuten R1, R3 und $R_4$ Wasserstoff oder Methyl und $R_2$ Wasserstoff oder Methoxy.

Eine weitere bevorzugte Gruppe innerhalb der Verbindungen der allgemeinen Formel 1 ist jene, in der n die Zahl 1 bedeutet.

Die Verbindungen der allgemeinen Formel 1 können erfindungsgemäß dadurch hergestellt werden, daß man

a)eine Verbindung der allgemeinen Formel

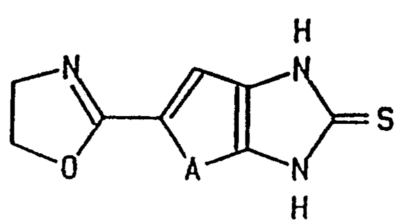

II

worin A die in Formel 1 angegebene Bedeutung hat, mit einer Verbindung der allgemeinen Formel

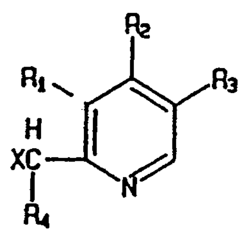

HX

III

worin X für Chlor oder Brom steht und
$R_1$,$R_2$,$R_3$ und $R_4$ die in Formel 1 angegebene Bedeutung haben, in Gegenwart von mindestens 2 Äquivalenten einer starken Base umsetzt, worauf man
b)gegebenfalls die so erhaltenen Verbindungen der allgemeinen Formel 1, worin n = O bedeutet, mit äquivalenten Mengen einer organischen Persäure oder Wasserstoff-peroxid zu einer Verbindung der allgemeinen Formel 1 umsetzt, in der n die Zahl 1 bedeutet.

Die Umsetzung nach Verfahrensschritt a) wird vorteilhafterweise so durchgeführt, daß man die Verbindungen der Formel 11 und 111 in einem inerten organischen Lösungsmittel, beispielsweise in einem niedersiedenden aliphatischen Alkohol, wie Methanol, Ethanol, Isopropanol und dergleichen, vorzugsweise in Methanol, suspendiert, wobei man aus Gründen der besseren Aufarbeitbarkeit die Verbindungen der Formel 11 zweckmäßigerweise im Überschuß einsetzt und dann mindestens 2 Äquivalente einer starken Base, vorzugsweise NaOH oder KOH, gelöst in etwas Wasser, bei Raumtemperatur zutropfen läßt.
Die Reaktionszeit beträgt, abhängig von den Ausgangsverbindungen, dem Lösungsmittel und der Temperatur etwa 2 - 8 Stunden.

Gemäß Verfahrensschritt b) können die Sulfoxidverbindungen, in denen n in der allgemeinen Formel 1 die Zahl 1 bedeutet, ausgehend von den nach Verfahrensschritt a) erhaltenen Sulfidverbindungen mit der Bedeutung für n = 0 durch partielle Oxidation mit geeigneten Oxidationsmitteln erhalten werden. Als solche Oxidationsmittel werden vorzugsweise etwa äquivalente Mengen einer organischen Persäure wie Peressigsäure, Perbenzoesäure oder m-Chlorperbenzoesäure, in einem reaktionsinerten Lösungsmittel, z.B. Methylenchlorid oder Chloroform, bei Temperaturen zwischen etwa -10 °C und -50 °C , oder etwa äquivalente Mengen 30 proz. $H_2O_2$ in Eisessig bei Raumtemperatur verwendet.

Die Verbindungen der allgemeinen Formel 1 unterliegen der Tautomerie und können daher auch in allen zu Formel 1 tautomeren Formen vorliegen.

Die Ausgangsverbindungen der allgemeinen Formel 111 sind bekannt. Die für das Verfahren verwendeten Ausgangverbindungen der allgemeinen Formel 11 können ausgehend von bekannten Produkten in an sich bekannter Weise hergestellt werden. Insbesondere können sie gemäß Reaktionsschema 1 und gemäß den spezifischen Angaben in den Beispielen synthetisiert werden. Die im Reaktionsschema angegebenen Startverbindungen der Formeln IV (FLUKA Art-Nr. 25450) und IX (C.D. HURD und K.L.KREUZ, J.Am. Chem. Soc. 74 ,2965(1952) sind literaturbekannt.

Reaktionsschema

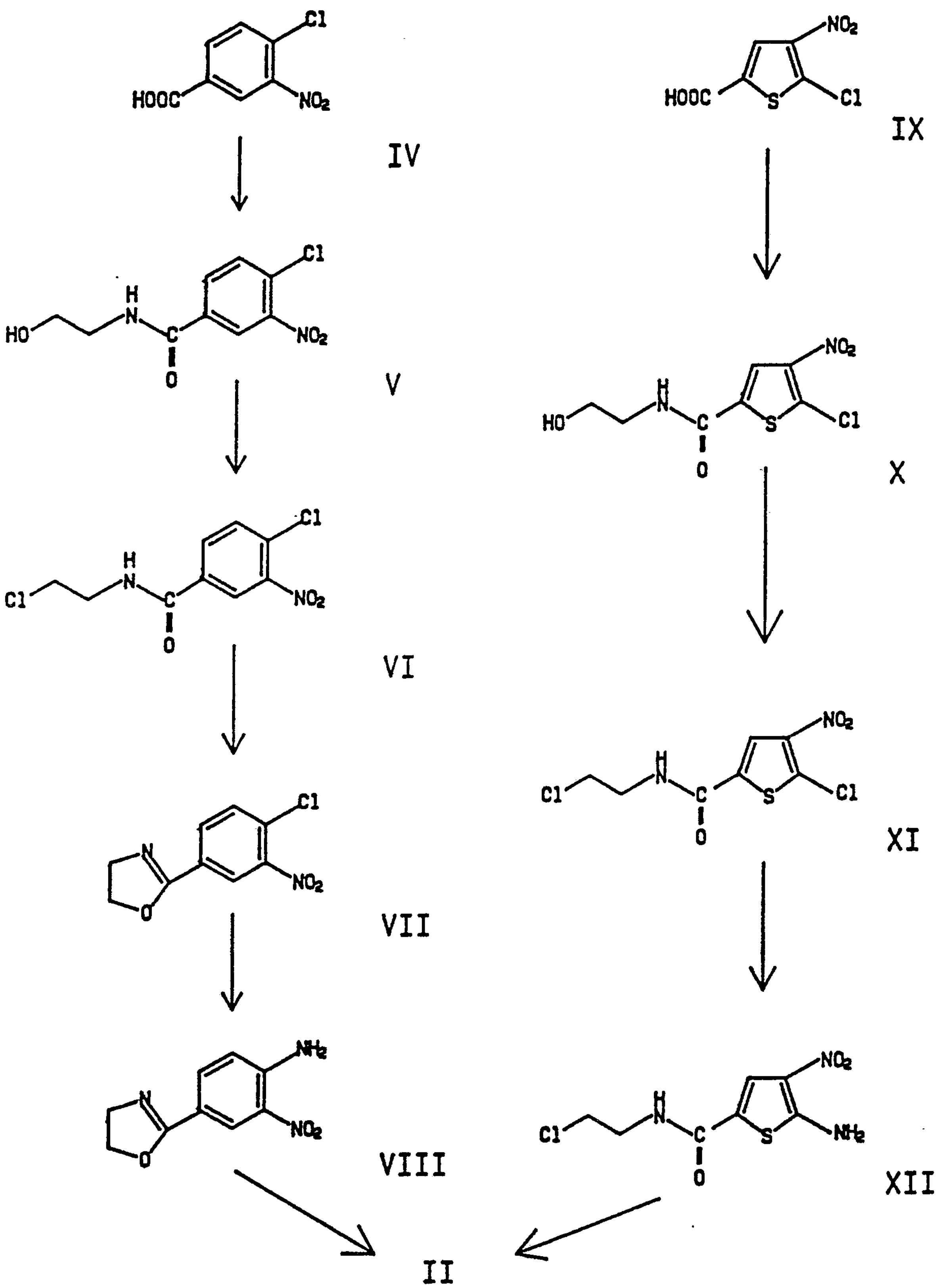

IV

V

VI

VII

VIII

IX

X

XI

XII

II

Die Verbindungen der allgemeinen Formel I besitzen wertvolle pharmakologische Eigenschaften. Insbesondere bewirken sie eine Blockade der ($H^+$ + $K^+$)-ATPase und können daher beispielsweise zur Behandlung oder Prophylaxe von Magen- und Zwölffingerdarmgeschwüren und anderen durch erhöhte Magensäuresekretion verursachten Erkrankungen in der Humanmedizin eingesetzt werden. Sie haben sich im Enzymtest als ca. fünfmal so wirksam wie Omeprazol, der bevorzugten Substanz der EP 5129, erwiesen. Die Verbindungen der allgemeinen Formel I können als Heilmittel, z.B. in Form pharmazeutischer Präparate Verwendung finden, welche die erfingungsgemäßen Verbindungen in Mischung mit einem für die enterale oder parenterale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline oder dergleichen enthalten. Die pharmazeutischen Präparate können in fester Form z.B. als Tabletten, Dragees, Suppositorien, Kapseln, oder in flüssiger Form z.B. als Lösungen, Suspensionen oder Emulsionen vorliegen. Gegebenfalls sind sie sterilisiert und/oder enthalten Hilfsstoffe, wie Konservierungs-, Stabilisierungs- oder Emulgiermittel, Salze zur Veränderung des osmotischen Druckes oder Puffer. Sie können auch in Kombination mit anderen therapeutisch wertvollen Stoffen verabreicht werden.

Beispiel 1:

5-(4,5-Dihydro-2-oxazolyl)-2-((3,5-dimethyl-4-methoxy2-pyridyl)-,m thylthio) -1H-benzimidazol
(Formel I: A = CH=CH, $R_1$ und $R_3$ = $CH_3$, $R_2$ = $OCH_3$, $R_4$ = H, n = 0)

6,00 g (27,4 mmol) 5-(4-5-Dihydro-2-oxazolyl)-1,3-dihydro-benzimidazol-2-thion (Formel II: A = CH=CH) und 6,08 g (27,4 mmol) 2-Chlormethyl-4-methoxy-3,5-dimethylpyridin-Hyrochlorid werden in 120 ml Methanol suspendiert und innerhalb von 12 Minuten 27,8 ml (55,6 mmol) 2n wäßrige NaOH zugetropft. Dabei steigt die Temperatur auf 30 °C. Es wird noch 3 Stunden bei Raumtemperatur gerührt, zur Trockene eingedampft und der Rückstand in 140 ml Wasser aufgenommen. Durch Zugabe von Eisessig wird auf pH = 4,5 angesäuert und dreimal mit je 200 ml Chloroform extrahiert. Die vereinigten organischen Phasen werden über Natriumsulfat/Aktivkohle getrocknet, filtriert und eingedampft. Der Rückstand wird mit Acetonitril digeriert, die Kristalle abgesaugt und bei 50 °C/20 mbar getrocknet. Zur weiteren Reinigung wird aus Aceton umkristallisiert.
Ausbeute: 5,6 g gelbliche Kristalle (55,5 % d. Th.)
Fp = 188-190 °C (Aceton)
Das Ausgangsmaterial kann wie folgt hergestellt werden:

4-Chlor-N-(2-hydroxyethyl)-3-nitro-benzoesäureamid (Formel V des Reaktionsschemas)

64,4 g (0,319 mol) 4-Chlor-3-nitro-benzoesäure werden in 400 ml Thionylchlorid eingerührt und 3 Stunden unter Rückfluß erhitzt. Anschließend wird eingedampft und der Rückstand in 300 ml abs. Methylenchlorid gelöst. Diese Lösung wird bei -10 °C zu einer Lösung von 40,9 g (0,670 mol) Ethanolamin in 400 ml abs. Methylenchlorid innerhalb von 2 Stunden zugetro pft. Es wird noch 30 Minuten gerührt, der Niederschlag abgesaugt, zweimal mit je 100 ml 0,25n HCl und einmal mit 100 ml Wasser gewaschen und bei 50 °C/20 mbar getrocknet.
Ausbeute: 83,1 g hellgelbes Rohprodukt, enthält 1 Mol Kristallwasser (99 % d. Th.)
Fp = 130-133 °C (Dioxan)

4-Chlor-N-(2-chlorethyl)-3-nitro-benzoesäureamid (Formel VI des Reaktionsschemas)

101,0 g (0,385 mol) 4-Chlor-N-(2-hydroxyethyl)-3-nitro-benzoesäureamid. $H_2O$ werden portionsweise in 450 ml Thionylchlorid eingerüht. Es wird noch 30 Minuten bei Raumtemperatur gerührt und anschließend im Vakuum eingedampft.
Ausbeute: 99,1 g gelbliche Kristalle (98 % d.Th.)
Fp = 94-96 °C (Dioxan)

2-(4-Chlor-3-nitrophenyl)-4,5-dihydro-oxazol (Formel VII des Reaktionsschemas)

99,0 g (0376 mol) 4-Chlor-N-(2-chlorethyl)-3-nitrobenzoesäureamid werden in 800 ml Ethanol unter Rühren auf 70 °C erhitzt und 217 ml (0,434 mol) 2n wäßrige NaOH zugegeben. Es wird noch 30 Minuten bei 70 °C gerührt. Danach wird eingedampft und der Rückstand in Wasser aufgenommen. Es wird abgesaugt, mit Wasser gewaschen und bei 50 °C/20 mbar getrocknet.
Ausbeute: 74,2 g (87 % d.Th.)
Fp = 89-90 °C (Diisopropylether)

2-(4-Amino-3-nitro-phenyl)-4,5-dihydro-oxazol (Formel VIII des Reaktionsschemas)

10,0 g (44,1 mmol) 2-(4-Chlor-3-nitro-phenyl)-4,5-dihydro-oxazol werden in einem Stahlautoklaven in 60 ml tert.-Butylamin 9 Stunden auf 125 °C erhitzt. Anschließend wird im Vakuum eingedampft und der trockene, feingepulverte Rückstand in 20 ml 85 proz. o-Phosphorsäure bei 80 °C eingerührt. Es wird 30 Minuten bei 80 °C gerührt. Anschließend wird abgekühlt, auf 125 ml Eiswasser geleert, mit 15 ml Ethylactat überschichtet, unter Eiskühlung mit Ammoniak auf pH = 8 gebracht, 10 Minuten heftig gerührt und der ausgefallene Niederschlag abgesaugt. Es wird mit Wasser und wenig Ethylacetat gewaschen und bei 50 °C/20 mbar getrocknet.
Ausbeute: 5,7 g gelbe Kristalle (62,3 % d.Th.)
Fp = 215-217 °C (Methanol)

5-(4,5-Dihydro-2-oxazolyl)-benzimidazol-2-thion
(II: A = CH=CH)

10,0 g (48,3 mmol) 2-(4-Amino-3-nitro-phenyl)-4,5-dihydro-oxazol werden in 80 ml Dioxan und 400 ml Methanol gelöst und in einer Mitteldruck-Hydrierapparatur mit $W_2$-Raney-Nickel bis zur berechneten Wasserstoffaufnahme bei Raumtemperatur hydriert. Der Katalysator wird über ein Filtrierhilfsmittel abgesaugt und das Filtrat zur Trockene eingedampft. Der Rückstand wird in 500 ml Ethanol gelöst, 9,4 g (72,2 mmol) Natriummethylxanthogenat zugesetzt und 3 Stunden unter Rückfluß erhitzt. Danach wird im Vakuum eingedampft, der Rückstand in 200 ml Wasser aufgenommen und durch Zugabe von 13 ml 2n NaOH gelöst. Es wird mit 2 Löffeln Aktivkohle versetzt, kurz gerührt und über ein Filtrierhilfsmittel abgesaugt. Das Filtrat wird mit Eisessig auf pH = 4,5 angesäuert, der ausgefallene Niederschlag abgesaugt und dreimal mit Wasser gewaschen. Es wird bei 60 °C/2O mbar getrocknet.
Ausbeute: 7,4 g bräunliche Kristalle (69,9 % d.Th.)
Fp = 265-268 °C (tlw. Zers. ab 200 °C)

Beispiel 2:

5-(4,5-Dihydro-2-oxazolyl)-2-((4-methoxy-2-pyridyl)methylthio)-1H-enzimidazol
(Formel I: A= CH=CH, $R_1$, $R_3$ und $R_4$ = H, $R_2$ = $OCH_3$, n = 0)

6,49 g(29,6 mmol) 5-(4,5-Dihydro-2-oxazolyl)-benzimidazol-2-thion (Formel II: A = CH=CH) und 5,46 g (28,1 mmol) 2-Chlormethyl-4-methoxypyridin,Hydrochlorid werden in 80 ml Methanol suspendiert und innerhalb von 12 Minuten 30 ml (60 mmol) 2n wäßrige NaOH zugetropft. Dabei steigt die Temperatur auf 30 °C. Es wird noch 6 Stunden bei Raumtemperatur gerührt, zur Trockene eingedampft und der Rückstand in 120 ml Wasser aufgenommen. Durch Zugabe von Eisessig wird auf pH = 4,5 angesäuert und dreimal mit je 200 ml Chloroform extrahiert. Die vereinigten organischen Phasen werden über Natriumsulfat/Aktivkohle getrocknet, filtriert und eingedampft. Der Rückstand wird mit Acetonitril kristalli siert, die Kristalle abgesaugt und bei 50 °C/20 mbar getrocknet. Zur weiteren Reinigung wird aus Aceton umkristallisiert.
Ausbeute: 5,60 g gelbliche Kristalle (58,5 % d.Th.)
Fp = 147-149 °C(Aceton)

Beispiel 3:

5-(4,5-Dihydro-2-oxazolyl)-2-((2-pyridyl)methylthio)-1H-benzimidaz l
(Formel I: A = CH=CH, $R_1$, $R_2$, $R_3$ und $R_4$ = H, n = 0)

6,00 g (27,4 mmol) 5-(4,5-Dihydro-2-oxazolyl)-1,3-dihydro-benzimidazol-2-thion (Formel II: A = CH=CH) und 4,49 g (27,4 mmol) 2-Chlormethylpyridin,Hydrochlorid werden in 120 ml Methanol suspendiert und innerhalb von 10 Minuten 27,5 ml (54,7 mmol) 2n wäßrige KOH zugetropft, wobei die Temperatur auf 28 °C ansteigt. Es wird 8 Stunden bei Raumtemperatur gerührt, danach das Lösungsmittel abdestilliert und der Rückstand in 120 ml Wasser aufgenommen. Es wird mit Eisessig auf pH = 4,5 angesäuert und dreimal mit insgesamt 350 ml Methylenchlorid ausgeschüttelt. Die vereinigten organischen Phasen werden über Natriumsulfat/Aktivkohle getrocknet, filtriert und eingedampft. Das verbleibende dunkelbraune Öl wird mit Acetonitril kristallisiert und die Kristallisation über Nacht im Tiefkühlschrank vervollständigt. Die Kristalle werden abgesaugt, mit wenig kaltem Acetonitril gewaschen und bei 50 °C/20 mbar getrocknet.
Ausbeute: 5,70 g beige Kristalle (67,1 % d.Th.)
Fp = 178-181 °C (Aceton)

Beispiel 4:

5-(4,5-Dihydro-2-oxazolyl)-2-((3,5-dimethyl-4-methoxy2-pyridyl)met ylsulfinyl)-1H-benzimidazol
(Formel I: A = CH=CH, $R_1$ und $R_3$ = $CH_3$, $R_2$ = $OCH_3$, $R_4$ = H, n = 1)

Zu einer Lösung von 6,5 g (17,6 mmol) 5-(4,5-Dihydro-2-oxazolyl)-2-((3,5-dimethyl-4-methoxy-2-pyridyl)methylt io)-1H-benzimidazol in 200 ml Chloroform wird unter Rühren bei einer Raumtemperatur zwischen -12 ° und -10 °C eine Lösung von 3,65 g(18,0 mmol) 85 proz. 3-Chlorperbenzoesäure in 65 ml Chloroform innerhalb von 30 Minuten zugetropft. Es wird noch 1 Stunde bei -10 °C gerührt und anschließend zweimal mit 40 ml ges. Natriumhydrogencarbonatlösung extrahiert. Die wäßrigen Phasen werden einmal mit 50 ml Chloroform gewaschen und die vereinigten organischen Phasen über Natriumsulfat unter Zusatz von Aktivkohle getrocknet, filtriert und eingedampft. Der Rückstand wird mit Ethanol kristallisiert und aus Ethanol unter Zusatz von Aktivkohle umkristallisiert.
Ausbeute: 3,05 g gelbliche Kristalle (45,0 % d.Th.)
Fp = 112-114 °C zers. (Ethanol)

Beispiel 5:

5-(4,5-Dihydro-2-oxazolyl)-2-((3,5-dimethyl-4-methoxy-2-pyridyl)-m thylthio)-3H-thieno (2,3-d)imidazol
(Formel I: A = S, $R_1$ und $R_3$ = $CH_3$, $R_2$ = $OCH_3$, $R_4$ = H, n = 0)

5,00 g (22,2 mmol) 5-(4,5-Dihydro-2-oxazolyl)-1,3-dihydro-thieno-(2,3-d)imidazol-2-thion (Formel II: A = S) und 3,45 g (15,5 mmol) 2-Chlormethyl-4-methoxy-3,5-dimethylpyridin-Hydrochlorid werden in 70 ml Methanol suspendiert und innerhalb von 10 Minuten 20 ml (40 mmol) 2n wäßrige NaOH zugetropft. Dabei steigt die Temperatur auf 32 °C. Es wird noch 3 Stunden bei Raumtemperatur gerührt, zur Trockene eingedampft und der Rückstand in 100 ml Wasser und 2 ml Eisessig aufgenommen. Es wird dreimal mit je 70 ml Chloroform extrahiert. Die vereinigten organischen Phasen werden über Natriumsulfat/Aktivkohle getrocknet, filtriert und eingedampft. Der Rückstand wird mit Acetonitril digeriert, die Kristalle abgesaugt und bei 50 °C/20 mbar getrocknet.
Zur weiteren Reinigung wird neuerlich aus Acetonitril umkristallisiert.
Ausbeute: 4,56 g gelbliche Kristalle (78,4 % d.Th.)
Fp = zers. ab 191 °C (Acetonitril)

Das Ausgangsmaterial kann wie folgt hergestellt werden:

5-Chlor-N-(2-hydroxyethyl)-4-nitro-2-thiophencarbonsäureamid (Formel X des Reaktionsschemas)

36,9 g (0,178 mol) 5-Chlor-4-niro-2-thiophencarbonsäure werden in 190 ml Thionylchlorid eingerührt und 3 Stunden unter Rückfluß erhitzt.

Anschließend wird überschüssiges Thionylchlorid im Vakuum abdestilliert. Der Rückstand wird in 300 ml abs. Methylenchlorid gelöst und diese Lösung bei -15 °C zu einer Lösung von 23,2 g (0,380 mol) Ethanolamin in 120 ml abs. Methylenchlorid innerhalb von 2 Stunden zugetropft. Es wird noch 1 Stunde gerührt, der ausgefallene Niederschlag abgesaugt, einmal mit 100 ml 0,25n HCl und einmal mit 100 ml Wasser gewaschen und bei 40 °C/20 mbar getrocknet.
Ausbeute: 35,7 g hellgelbe Kristalle (80, 1 % d.Th.)
Fp = 136-138 °C (Dioxan)

5-Chlor-N-(2-chlorethyl)-4-nitro-2-thiophencarbonsäureamid (Formel XI des Reaktionsschemas)

In 180 ml Thionylchlorid werden bei Raumtemperatur 35,5 g(0,142 mol) 5-Chlor-N-(2-hydroxyethyl)-4-nitro-2-thiophencarbonsäureamid portionsweise eingerührt. Nach 90 Minuten Rühren bei Raumtemperatur wird im Vakuum eingedampft und bei 40 °C/20 mbar getrocknet.
Ausbeute: 37,5 g hellgelbe Kristalle (98,4 % d.Th.)
Fp = 111-114 °C (Benzol)

5-Amino-N-(2-chlorethyl)-4-nitro-2-thiophencarbonsäureamid (Formel XII des Reaktionsschemas)

In eine Lösung von 30,0 g (0,111 mol) 5-Chlor-N-(2-chlorethyl)-4-nitro-2-thiophencarbonsäureamid in 300 ml abs. Dioxan wird unter Rühren Ammoniakgas eingeleitet. Nach Abklingen der zuerst exothermen Reaktion wird noch 9 Stunden unter weiterem Gaseinleiten gerührt und anschließend das Reaktionsgemisch im Vakuum eingedampft. Der Rückstand wird zwischen Wasser und Ethylacetat verteilt und die Phasen getrennt. Die wäßrige Phase wird noch fünfmal mit je 100 ml Ethylacetat extrahiert, die vereinigten organischen Phase über Natriumsulfat unter Zusatz von Aktivkohle getrocknet, filtriert und eingedampft.
Ausbeute: 25,7 g gelbe Kristalle (92,3 % d.Th.)
Fp = 2O2-208 °C (Acetonitril)

5-(4,5-Dihydro-2-oxazoly)-1,3-dihydro-thieno)2,3-d)imidazol-2-thio (II: A = S)

10,0 g (40,1 mmol) 5-Amino-N-(2-chlorethyl)-4-nitro-2-thiophencarbonsäureamid werden in 100 ml Dioxan und 100 ml Methanol gelöst und in einer Mitteldruck-Hydrierapparatur mit $W_2$-Raney-Nickel als Katalysator bis zur berechneten Wasserstoffaufnahme bei Raumtemperatur hydriert. Der Katalysator wird über ein Filtrierhilfsmittel abgesaugt und 6,3 g (48,0 mmol) Natriummethylxanthogenat zugesetzt und 2,5 Stunden unter Rückfluß erhitzt. Danach wird das Reatkionsgemisch eingedampft, der Rückstand in 200 ml Wasser und 10 ml ges. Natriumcarbonatlösung aufgenommen. Es wird von wenig unlöslichem Rückstand abfiltriert und mit Eisessig auf pH = 4,5 angesäuert, der ausgefallene Niederschlag abgesaugt und dreimal mit Wasser gewaschen. Es wird bei 60 °C/20 mbar getrocknet.
Ausbeute: 3,3 g grünliche Kristalle (36,4 % d.Th.)
Fp 300 °C (tlw. Zers.)

Beispiel 6:

5-(4,5-Dihydro-2-oxazolyl)-2-((4-methoxy-2-pyridyl)methylthio)-3H-hieno(2,3-d)imidazol
(Formel I: A = S, $R_1$, $R_3$ und $R_4$ = H, $R_2$ = $OCH_3$, n = 0)

2,70 g(12,0 mmol) 5-(4,5-Dihydro-2-oxazoly)-1,3-dihydro-thieno-(2,3-d)imidazol-2-thion (Formel II: A = S) und 2,09 g (10,8 mmol) 2-Chlormethyl-4-methoxypyridin-Hydrochlorid werden in 40 ml Methanol suspendiert und innerhalb von 15 Minuten 13 ml (26,0 mmol) 2n wäßrige KOH zugetropft, wobei die Temperatur auf 28 °C ansteigt. Es wird noch 7 Stunden bei Raumtemperatur gerührt, zur Trockene eingedampft und der Rückstand in 50 ml Wasser aufgenommen und mit Eisessig auf pH = 4,5 angesäuert. Es wird dreimal mit insgesamt 150 ml Chloroform exrahiert. Die vereinigten organischen Phasen werden über Natriumsulfat/Aktivkohle getrocknet, filtriert und eingedampft. Der Rückstand wird mit Acetonitril digeriert, die Kristalle abgesaugt und bei 50 °C/20 mbar getrocknet.

Zur weiteren Reinigung wird neuerlich aus Acetonitril umkristallisiert.
Ausbeute: 2,33 g gelbliche Kristalle (62,4 % d.Th.)
Fp = zers. ab 182 °C (Acetonitril)

Beispiel 7:

5-(4,5-Dihydro-2-oxazolyl)-2-((2-pyridyl)methylthio)-3H-thieno-(2, -d)imidazol
(Formel I: A = S, $R_1$, $R_2$, $R_3$ und $R_4$ = H, n = 0)

1,50 g (6,66 mmol) 5-(4,5-Dihydro-2-oxazolyl)-1,3-dihydro-thieno-(2,3-d)imidazol-2-thion (Formel II: A = S) und 0,76 g (4,63 mmol) 2-Chlormethylpyridin,Hydrochlorid werden in 30 ml Methanol suspendiert und innerhalb von 15 Minuten 7 ml (14 mmol) 2n wäßrige NaOH zugetropft. Dabei steigt die Temperatur auf 31 °C. Nach 2 Stunden Rühren bei Raumtemperatur wird eingedampft, der Rückstand in 60 ml Wasser und 1,5 ml Eisessig aufgenommen und dreimal mit je 50 ml Mehtylenchlorid extrahiert. Die vereinigten organischen Phasen werden über Natriumsulfat/Aktivkohle getrocknet, filtriert und eingedampft. Der Rückstand wird mit Acetonitril digeriert, die Kristalle abgesaugt und bei 50 °C/20 mbar getrockenet.
Zur weiteren Reinigung wird neuerich aus Acetonitril umkristallisiert.
Ausbeute: 1,28 g gelbliche Kristalle (87,3 % d.Th.)
Fp = zers. ab 168 °C (Acetonitril)

Beispiel 8:

5-(4,5-Dihydro-2-oxazolyl)-2-((3,5-dimethyl-4-methoxy-2-pyridyl)-m thylsulfinyl)-3H-thieno(2,3-d)imidazol
(Formel I: A = S, $R_1$ und $R_3$ = $CH_3$, $R_2$ = $OCH_3$, $R_4$ =H, n = 1)

Zu einer Lösung von 3,0 g (8,01 mmol) 5-(4,5-Dihydro-2-oxazolyl)-2-((3,5-dimethyl-4-methoxy-2-pyridyl)methylt io)-3H-thieno-(2,3-d)imidazol in 100 ml Chloroform wird unter Rühren bei einer Temperatur zwischen -25 ° und -20 °C eine Lösung von 1,63 g (8,01 mmol) 85 proz. 3-Chlorperbenzoesäure in 40 ml Chloroform innerhalb von 30 Minuten zugetropft. Es wird noch 1 Stunde bei -20 °C gerührt und anschließend zweimal mit 20 ml ges. Natriumhydrogencarbonatlösung extrahiert. Die wäßrigen Phasen werden einmal mit 50 ml Chloroform gewaschen und die vereinigten organischen Phasen über Natriumsulfat unter Zusatz von Aktivkohle getrocknet, filtriert und eingedampft. Der Rückstand wird mit Acetonitril kristallisiert und aus Ethanol unter Zusatz von Aktivkohle umkristallisiert.
Ausbeute: 2,80 g gelbliche Kristalle (89,5 % d.Th.)
Fp = zers. ab 131 °C

Beispiel 9:

5-(4,5-Dihydro-2-oxazolyl)-2-((2-pyridyl)methylsulfinyl)-3H-thieno 2,3-d)imidazol
(Formel I: A = S, $R_1$, $R_2$, $R_3$ und $R_4$ = H, n = 1)

1,00 g (3,16 mmol) 5-(4,5-Dihydro-2-oxazolyl)-2-((2-pyridyl)-methylthio)-3H-thieno (2,3-d)imidazol werden in 15 ml Eisessig aufgenommen und bei 5 ° - 10 °C mit 0,36 g (3,16 mmol) 30 proz. $H_2O_2$ tropfenweise versetzt. Es wird noch 30 Minuten bei Raumtemperatur gerührt, mit 100 ml Wasser verdünnt und dreimal mit je 30 ml Methylenchlorid extrahiert. Die vereinigten organischen Phasen werden mit Natriumhydrogencarbonatlösung gewaschen, über Natriumsulfat getrocknet, filtriert und eingedampft.
Ausbeute: 0,64 g gelbliche Kristalle (60,9 % d.Thg.)
Fp = zers. ab 190 °C

Beispiel 10:

In einem Vergleichsversuch wurde die Hemmwirkung von Omeprazol (der bevorzugten Verbindung der EP 5129) und derVerbindung des Beispiels 4 der vorliegenden Anmeldung (Substanz A) auf $H^+/K^+$-ATPase untersucht und die $IC_{50}$ berechnet. Hierbei wurden folgende Werte gefunden:

| | Hemmung der $H^+/K^+$-ATPase $IC_{50}$ (uMOL) |
|---|---|
| Omeprazol | 28.0 |
| Substanz A | 5,3 |

Dieser Vergleich zeigt, daß sie Substanz A die $H^+/K^+$-ATPase ca. fünfmal stärker hemmt als Omeprazol.

## Ansprüche

1. 4,5 -Dihydro-oxazole der allgemeinen Formel

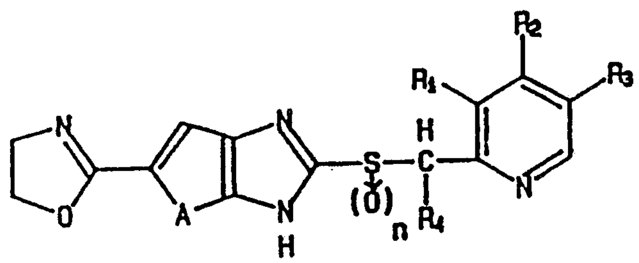

I

in der
A -CH=CH- oder -S-,
$R_1$, $R_3$ und $R_4$ unabhängig voneinander Wasserstoff oder $(C_1-C_4)$-Alkyl,
$R_2$ Wasserstoff oder (C1-C4)-Alkoxy und
n O oder 1 bedeuten

2. Verbindungen der in Anspruch 1 definierten allgemeinen Formel 1, in der n die Zahl 1 bedeutet.

3. Verbindungen der allgemeinen Formel 1 nach einem der Ansprüche 1 oder 2, worin
$R_1$, $R_3$ und $R_4$ Wasserstoff oder Methyl und
$R_2$ Wasserstoff oder Methoxy bedeuten.

4. Verfahren zur Herstellung von Verbindungen der in Anspruch 1 definierten allgemeinen Formel

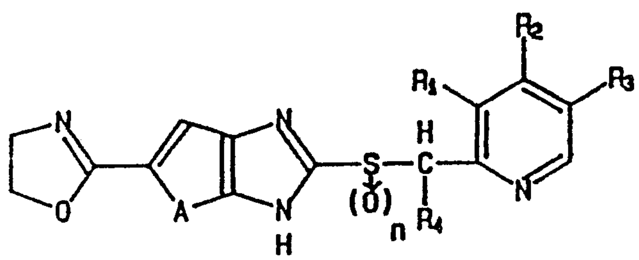

I

dadurch gekennzeichnet, daß man
a)eine Verbindung der allgemeinen Formel

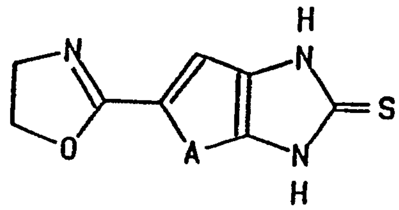

II

worin A die in Formel 1 angegebene Bedeutung hat, mit einer Verbindung der allgemeinen Formel

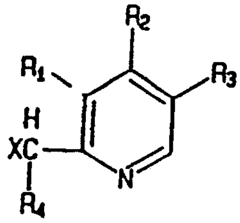

HX

III

worin X für Chlor oder Brom steht und $R_1$, $R_2$, $R_3$ und $R_4$ die in Formel 1 angegebene Bedeutung haben, in Gegenwart von mindestens 2 Äquivalenten einr starken Base umsetzt, worauf man b)gegebenenfalls die so erhaltenen Verbindungen der allgemeinen Formel I, worin n = 0 bedeutet, mit äquivalenten Mengen einer organischen Persäure oder Wasserstoffperoxid zu einer Verbindung der allgemeinen Formel 1 umsetzt, in der n die Zahl 1 bedeutet.

**5.** Pharmazeutische Präparate, enthaltend Verbindungen der allgemeinen Formel 1 des Formelblattes nach Anspruch 1 in Kombination mit üblichen Hilfs-und Trägerstoffen.

**6.** Verbindungen nach Anspruch 1 zur Verwendung als Wirkstoffe für Arzneimittel zur Behandlung und Prophylaxe von Krankheiten, die durch erhöhte Magensäuresekretion hervorgerufen werden, wie Magen- und Zwölffinger-darmgeschwüre.

Patentansprüche für folgende Vertragsstaaten: AT, ES, GR

**1.** Verfahren zur Herstellung neuer Derivate von 4,5-Dihydro-oxazolen der allgemeinen Formel

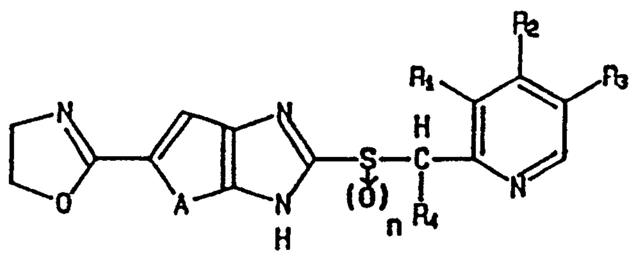

I

in der
A -CH = CH- oder -S-,
$R_1$,$R_3$ und $R_4$ unabhängig voneinander Wasserstoff oder $(C_1-C_4)$-Alkyl
$R_2$ Wasserstoff oder $(C_1-C_4)$-Alkoxy und
n 0 oder 1 bedeuten,
dadurch gekennzeichnet, daß man
a)eine Verbindung der allgemeinen Formel

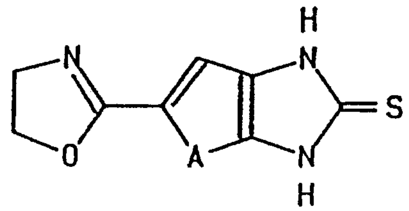 II

worin A die in Formel 1 angegebene Bedeutung hat, mit einer Verbindung der allgemeinen Formel

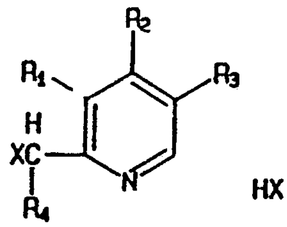 III

worin X für Chlor oder Brom steht und

$R_1$, $R_2$, $R_3$ und $R_4$ die in Formel 1 angegebene Bedeutung haben, in Gegenwart von mindestens zwei Äquivalenten einer starken Base umsetzt, worauf man

b) gegebenfalls die so erhaltenen Verbindungen der allgemeinen Formel 1, worin n = 0 bedeutet, mit äquivalenten Mengen einer organischen Persäure oder Wasserstoffperoxid zu einer Verbindung der allgemeinen Formel 1 umsetzt, in der n die Zahl 1 bedeutet.

**2.** Verfahren nach Anspruch 1, dadurch gekennzeichnet, daß man die Umsetzung in Stufe a) in einem niedrigsiedendem Alkohol, vorzugsweise Methanol, durchführt.

**3.** Verfahren nach einem der Ansprüche 1 oder 2, dadurch gekennzeichnet, daß man in Stufe a) die Verbindung der allgemeinen Formel 11 des Formelblattes im Überschuß einsetzt.

**4.** Verfahren nach einem der Ansprüche 1 bis 3, dadurch gekennzeichnet, daß man die Oxydation in Stufe b) mit äquivalenten Mengen Peressigsäure, Perbenzoesäure oder m-Chlorperbenzoesäure durchführt.

**5.** Verfahren nach Anspruch 4, dadurch gekennzeichnet, daß man die Oxidation in Stufe b) in Methylenchorid oder Chloroform bei Temperaturen zwischen - 10° C und -50°C durchführt.

**6.** Verfahren nach Anspruch 4, dadurch gekennzeichnet, daß man die Oxidation in Stufe b) mit äquivalenten Mengen Wasserstoffperoxid durchführt.

**7.** Verfahren nach Anspruch 6, dadurch gekennzeichnet, daß man die Oxidation in Stufe b) mit 30%igem Wasserstoffperoxid in Eisessig durchführt.

## Claims

**1.** 4,5-Dihydrooxazole of the general formula

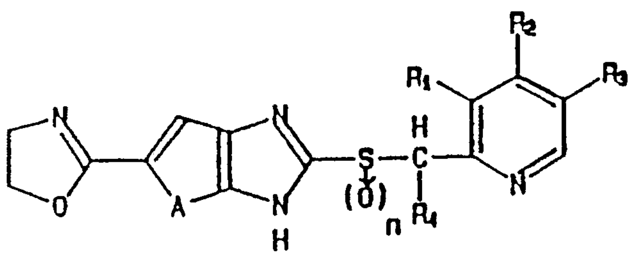 I

in which

A denotes -CH=CH- or -S-,
$R_1$, $R_3$ and $R_4$, independently of one another, denote hydrogen or ($C_1$-$C_4$)-alkyl,
$R_2$ denotes hydrogen or ($C_1$-$C_6$)-alkoxy, and
n denotes U or 1.

2. Compound of the general formula I defined in Claim 1, in which n denotes the number 1.

3. Compound of the general formula I according to either of Claims 1 or 2, in which
$R_1$, $R_3$ and $R_4$ denote hydrogen or methyl, and
$R_2$ denotes hydrogen or methoxy.

4. Process for the preparation of a compound of the general formula I defined in Claim 1

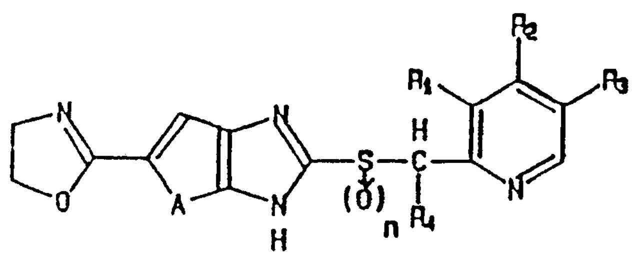

I

characterized in that
a) a compound of the general formula

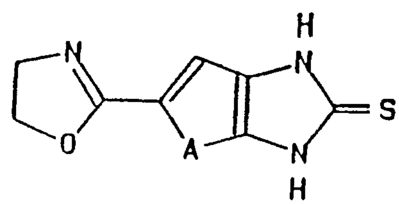

II

in which A has the meaning given in formula I, is reacted with a compound of the general formula

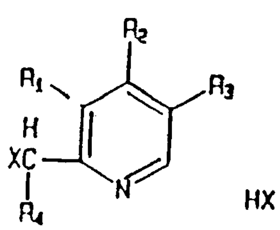

III

in which X represents chlorine or bromine, and $R_1$, $R_2$, $R_3$ and $R_4$ have the meaning given in formula I, in the presence of at least 2 equivalents of a strong base, after which
b) the compound thus obtained of the general formula I in which n denotes 0 is reacted, if appropriate, with equivalent amounts of an organic peracid or hydrogen peroxide to form a compound of the general formula I in which n denotes the number 1.

5. Pharmaceutical preparation which contains a compound of the general formula I of the sheet of formulae according to Claim 1 together with conventional adjuvants and excipients.

6. Compound according to Claim 1 for use as an active ingredient in medicaments for treatment and prophylaxis of disorders which are caused by increased gastric secretions, such as gastric and duodenal ulcers.

Claims for the following Contracting States: AT, ES, GR

1. Process for the preparation of novel derivatives of 4,5-dihydrooxazoles of the general formula

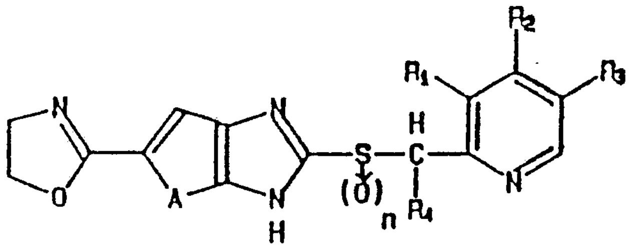

I

in which
A denotes -CH=CH- or -S-,
$R_1$, $R_3$ and $R_4$, independently of one another, denote hydrogen or ($C_1$-$C_4$)-alkyl,
$R_2$ denotes hydrogen or ($C_1$-$C_4$)-alkoxy, and
n denotes 0 or 1 characterized in that
a) a compound of the general formula

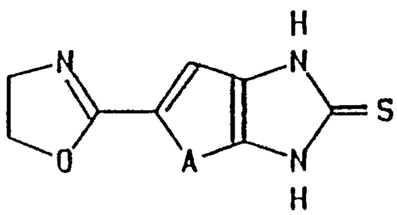

II

in which A has the meaning given in formula I, is reacted with a compound of the general formula

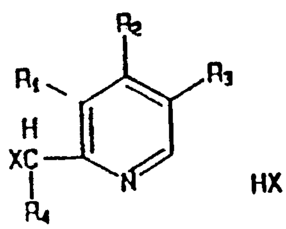

III

in which X represents chlorine or bromine, and
$R_1$, $R_2$ $R_3$ and $R_4$ have the meaning given in formula I, in the presence of at least two equivalents of a strong base, after which
b) the compound thus obtained of the general formula I in which n denotes 0 is reacted, if appropriate, with equivalent amounts of an organic peracid or hydrogen peroxide to form a compound of the general formula I in which n denotes the number 1.

2. Process according to Claim 1, characterized in that the reaction in step a) is carried out in a low-boiling alcohol, preferably methanol.

3. Process according to either of Claims 1 or 2, characterized in that, in step a), the compound of the general formula II of the sheet of formulae is employed in excess.

4. Process according to any one of Claims 1 to 3, characterized in that the oxidation in step b) is carried out using equivalent amounts of peracetic acid, perbenzoic acid or m-chloroperbenzoic acid.

5. Process according to Claim ,4, characterized in that the oxidation in step b) is carried out in methylene chloride or chloroform at temperatures between -10° C and -50° C.

6. Process according to Claim 4, characterized in that the oxidation in step b) is carried out using equivalent amounts of hydrogen peroxide.

7. Process according to Claim 6, characterized in that the oxidation in step b) is carried out using 30% strength hydrogen peroxide in glacial acetic acid.

## Revendications

1. 4,5-dihydro-oxazole de formule générale :

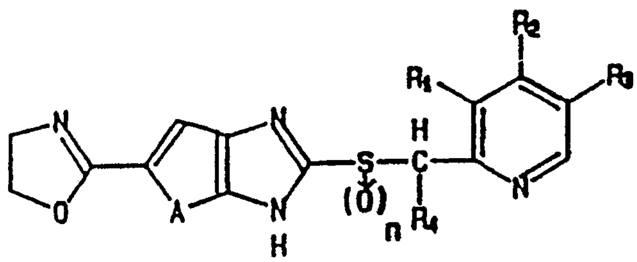

I

dans laquelle :
A signifie -CH=CH- ou -S-,
$R_1$, $R_3$ et $R_4$, indépendants les uns des autres, représentent l'hydrogène ou un groupe alkyle $C_1$-$C_4$,
$R_2$ représente l'hydrogéne ou un groupe alkoxy $C_1$-$C_4$ et n signifie 0 ou 1.

2. Composés selon la revendication 1, définis par la formule générale I dans laquelle n signifie le nombre 1.

3. Composés de formule générale I selon la revendication 1 ou 2, dans laquelle :
$R_1$, $R_3$ et $R_4$ signifient l'hydrogène ou un groupe méthyle, et $R_2$ représente l'hydrogène ou un groupe méthoxy.

4. procédé de préparation des composés définis dans la revendication 1 de formule générale :

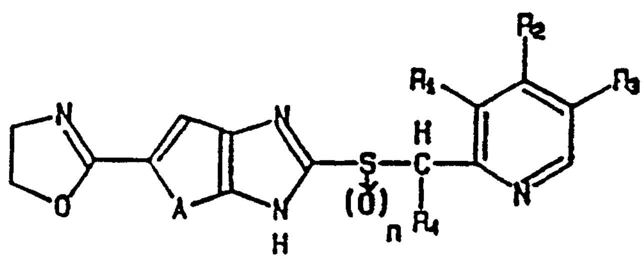

I

caractérisé en ce que :
a) on fait réagir un composé de formule générale

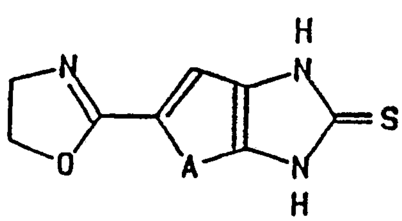

II

dans lequel A a la signification indiquée dans la formule I, avec un composé de formule générale :

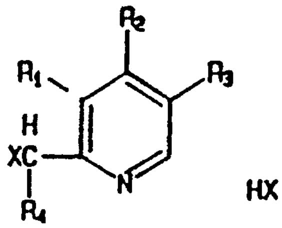

III

dans lequel X représente le chlore ou le brome7 $R_1$, $R_2$, $R_3$ et $R_4$ ont les significations indiquées dans la formule I, en présence d'au moins deux équivalents d'une base forte, et

b) on fait réagir le cas échéant les composés de formule générale I obtenus, où n = 0, avec les quantités équivalentes d'un peracide organique ou un peroxyde d'hydrogène pour obtenir un composé de formule générale 1, dans lequel n signifie le nombre 1.

**5.** Préparation pharmaceutique, renfermant les composés de formule générale I de la feuille de formules selon la revendication 1 en combinaison avec les adjuvants et substances vectrices habituelles.

**6.** Composés selon la revendication 1 pour l'utilisation à titre de principe actif dans des médicaments pour le traitement et la prophylaxie des maladies qui provoquent l'augmentation des sécrétions acides de l'estomac, comme les ulcères de l'estomac et du duodénum.

Revendications pour les Etats contractant suivants: AT, ES, GR

**1.** Procédé de préparation de nouveaux dérivés des 4,5-dihydrooxazoles de formule générale :

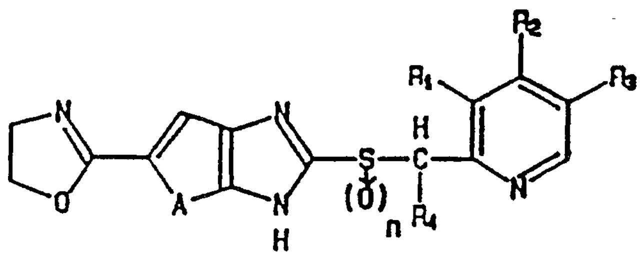

I

dans laquelle :

A signifie -CH = CH- ou -S-,

$R_1$, $R_3$ et $R_4$ indépendants les uns des autres, représentent l'hydrogéne ou un groupe alkyle $C_1$-$C_4$,

$R_2$ représente l'hydrogène ou un groupe alkoxy $C_1$-$C_4$ et n signifie 0 ou 1,

caractérisé en ce que :

a) on fait réagir un composé de formule générale

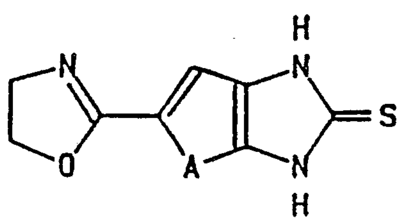

II

dans lequel A a la signification indiquée dans la formule I, avec un composé de formule générale :

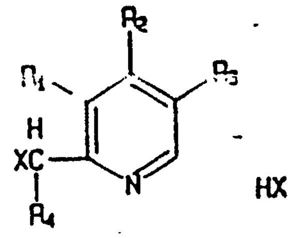

III

dans lequel X représente le chlore ou le brome, $R_1$, $R_2$,$R_3$ et $R_4$ ont les significations indiquées dans la formule I, en présence d'au moins deux équivalents d'une base forte, et

b) on fait réagir le cas échéant les composés de formule générale I obtenus, où n = 0, avec les quantités équivalentes d'un peracide organique ou un peroxyde d'hydrogène pour obtenir un composé de formule générale I, dans lequel n signifie le nombre 1.

**2.** Procédé selon la revendication 1, caractérisé en ce que la réaction suivant l'étape a) est conduite dans un alcool à bas point d'ébullition, tel le méthanol.

**3.** Procédé selon la revendication 1 ou 2, caractérisé en ce qu'on utilise dans l'étape a) le composé de formule générale II de la feuille de formules en excès.

**4.** Procédé selon l'une des revendications 1 à 3, caractérisé en ce qu'on réalise l'oxydation dans l'étape b) avec une quantité équivalente d'acide peracétique, perbenzoïque ou mchloroperbenzoïque.

**5.** Procédé selon la revendication 4, caractérisé en ce qu'on réalise l'oxydation dans l'étape b) dans le chlorure de méthylène ou le chloroforme à une température comprise entre -10 et -50 °C.

**6.** Procédé selon la revendication 4, caractérisé en ce que l'oxydation de l'etape b) est réalisée avec une quantité équivalente de peroxyde d'hydrogène.

**7.** Procédé selon la revendication 6, caractérisé en ce que l'oxydation de l'étape b) est réalisée avec une solution d'acide acétique à 30% de peroxyde d'hydrogène.